# EUROPEAN PATENT SPECIFICATION

(11) **EP 2 289 489 B1**
(45) Date of publication and mention of the grant of the patent: **16.07.2014**
(21) Application number: 10184394.4
(22) Date of filing: 05.11.2004
(51) Int. Cl.: A61K 9/08, A61K 31/13, A61P 25/00

(54) **Compositions comprising cyclohexylamines and aminoadamantanes**
Zusammensetzungen mit Cyclohexylaminen und Aminoadamantanen
Compositions comprenant des cyclohéxylamines et des aminoadamantanes

(30) Priority: 05.11.2003 US 517981 P
(43) Date of publication of application: 02.03.2011
(62) Divisional of application: 04810445.9
(73) Proprietor: Merz Pharma GmbH & Co. KGaA, 60318 Frankfurt (DE)
(72) Inventor: Dedhiya, Mahendra G., New York 10970 (US); Mahashabde, Shashank, New Jersey 08824 (US); Yang, Yan, New York 11577 (US); Goel, Anshu, New York 11756 (US); Seiller, Erhard, 61130 Nidderau (DE); Hauptmeier, Bernhard, 63571 Gelnhausen (DE)
(74) Representative: Goddard, Christopher Robert

(56) References cited:
- WO-A-03/040084
- US-A- 6 071 966

## Description

### FIELD OF THE INVENTION

The invention is directed to formulations of pharmaceutical compounds, such as the Cyclohexylamines and Aminoadamantanes which have antimicrobial properties. In particular, it is directed to aqueous based formulations with reduced amounts of preservatives which allow safe and convenient administration and flexible dosing and which, in the case of oral formulations, are easy to swallow. Optionally, the compositions contain components that provide the requisite stability and shelf life while reducing or avoiding incrustation of the composition around the container closure which leads to leaks and difficulty in opening the container.

### BACKGROUND OF THE INVENTION

Traditionally, pharmaceutical preparations are prepared in tablet form. In particular populations, such form is disadvantageous. For example, some patients may have difficulty with the fine motor skill required for administering oral forms and others may have difficulty swallowing an oral dosage form. Another problem may be that of administering an oral dosage form to non-compliant and/or combative patients. Pharmaceuticals are also available in liquid solution for oral administration. A liquid formulation has two major advantages over tablets: it allows flexible dosing, and it does not require the swallowing of solid dosage units, which may be difficult for many elderly patients. Flexible dosing, for example, may be recommended in the initial phase of therapy for some substances, where a starting dose is often a fraction of the regular dose. In the case of tablets, these have to be broken into halves for dose reduction, which again may be difficult for patients to do and may result in inconsistent dosing.

On the other hand, aqueous based formulations are also associated with certain disadvantages. One of the major drawbacks of multi-dose aqueous liquid compositions is their microbiological instability. When withdrawing a dose from a typical container, the remaining portion of the formulation is vulnerable to contamination with air-borne microbial organisms. After contamination, the formulation is liable to substantial microbial growth, in particular mold growth, but also yeast and bacteria growth. For this reason, multi-dose liquid or semi solid formulations are usually stabilized with one or two appropriate preservatives. In the case of multi-dose liquids, effective preservation is essential in terms of drug safety and stability. Oral liquids can also be formulated without preservatives, but in this case they must be kept under refrigeration and must be used within a short period of time, usually within a few days. In any event, if an aqueous formulation is designed for multiple use over a period of weeks or even months, it must incorporate a preservative to ensure microbiological stability.

Preservatives used in pharmaceutical compositions are usually regarded as safe in that they exhibit a low acute and chronic toxicity. However, preservatives have been associated with allergic and pseudoallergic reactions. For example, some people appear to be particularly sensitive to members of the paraben family (i.e. alkyl esters of p-hydroxybenzoic acid), which are also somewhat irritating to the skin and mucosae. Whenever possible, patients with such sensitivities should avoid contact with preservatives. Moreover, some less tolerable preservatives, such as certain nitrites, have been abandoned altogether.

Another problem associated particularly with oral aqueous formulations is the taste of the formulation. In order to mask a bitter taste, sweeteners are often added. Sweeteners, such as sugar or sorbitol, however, are known to crystallize around the container closure which causes it to "lock". These substances are deposited on the opening of the bottle and closure threads, subsequently drying and either preventing complete closure or preventing opening of the container. In an attempt to rectify this problem, solubilizers are added, however, they may contribute to ineffective closure due to the slickness of the solution, causing leakage upon transport or storage, particularly in inverted or side positions.

Clearly, there is a need for improved aqueous based pharmaceutical formulations, including formulations of Cyclohexylamines and Aminoadamantanes, which do not possess the disadvantages of existing formulations. In particular, there is a need for aqueous based formulations of Cyclohexylamines and Aminoadamantanes which are convenient, safe, tolerable and stable.

### SUMMARY OF THE INVENTION

We have discovered that Cyclohexylamines and Aminoadamantanes exhibit antimicrobial properties and consequently may be formulated as aqueous based pharmaceutical compositions, which are aqueous-based and free of preservatives, or which contain reduced amounts of preservatives, and which are therefore more tolerable to patients, in particular to those patients having a sensitivity to preservatives.

Specifically, the invention is directed to the use of a compound selected from neramexane, its optical isomers, diastereoisomers, polymorphs, enantiomers, hydrates, pharmaceutically acceptable salts and mixtures thereof as an antimicrobial compound in an aqueous based pharmaceutical composition comprising an aqueous vehicle, wherein the composition is preservative free or wherein the composition further comprises a preservative, wherein the concentration of the preservative is less than the concentration required to effectively preserve the corresponding placebo composition according to the USP <51> preservative efficacy test. Suitable compositions include aqueous liquid compositions for oral or parenteral use which comprise an NMDA receptor antagonist selected from neramexane, or a pharmaceutically acceptable salt such as neramexane mesylate. The compositions are further characterized in that they are substantially free of preservatives.

Further relevant compositions for use in the invention are for oral or parenteral use which comprise neramexane, or a pharmaceutically acceptable salt such as neramexane mesylate and at least one preservative, wherein the concentration of the preservative is less than the concentration required to effectively preserve the corresponding placebo composition.

The compositions of the invention can be conveniently presented in multiple-dose containers with reclosable closures to allow easy and flexible dosing and administration.

### DETAILED DESCRIPTION OF THE INVENTION

In accordance with the present invention, an aqueous liquid based pharmaceutical composition is provided for the administration of neramexane, its optical isomers, diastereomers, polymorphs, enantiomers, hydrates, pharmaceutically acceptable salts and mixtures thereof to a human or animal subject, where the composition includes neramexane, its optical isomers, diastereomers, polymorphs, enantiomers, hydrates, pharmaceutically acceptable salts and mixtures thereof and is in solution, suspension or gel form.

Representative compositions of the invention may be useful in the treatment of CNS diseases, including but not limited to the treatment of Alzheimer's disease (U.S. Patent Nos. 5,061,703 and 5,614,560) Parkinson's disease, AIDS dementia (U.S. Patent No. 5,506,231), neuropathic pain (U.S. Patent No. 5,334,618), epilepsy, glaucoma, hepatic encephalopathy, multiple sclerosis, stroke, depression (U.S. Patent No. 6,479,553), and tardive dyskinesia (Parsons et al., 1999), malaria, Borna virus, Hepatitis C (U.S. Patent Nos. 6,034,134, and 6,071,966). Additional pathologies are disclosed in U.S. Patent Nos. 5,614,560 and 6,444,702.

### ACTIVE PHARMACEUTICAL INGREDIENT

Use of a compound selected from neramexane, its optical isomers, diastereomers, polymorphs, enantiomers, hydrates, pharmaceutically acceptable salts and mixtures thereof as an antimicrobial compound in an aqueous based composition for pharmaceutical use comprising an aqueous vehicle wherein the composition is free of preservatives, or wherein the composition further comprises a preservative, wherein the concentration of the preservative is less than the concentration required to effectively preserve the corresponding placebo composition.

1-aminocyclohexane compounds used according to the invention are selected from the group consisting of:
1-amino-1,3,3,5,5-pentamethylcyclohexane (neramexane),
optical isomers, diastereomers, enantiomers, hydrates, pharmaceutically acceptable salts, and mixtures thereof.

Neramexane (1-amino-1,3,3,5,5-pentamethylcyclohexane) is disclosed, e.g., U.S. Patent No. 6,034,134.

According to the invention, neramexane may be applied as such or used in the form of its pharmaceutically acceptable salts. Suitable salts of the compound include, but are not limited to, acid addition salts, such as those made with hydrochloric, methylsulfonic, hydrobromic, hydroiodic, perchloric, sulfuric, nitric, phosphoric, acetic, propionic, glycolic, lactic pyruvic, malonic, succinic, maleic, fumaric, maleic, tartaric, citric, benzoic, carbonic cinnamic, mandelic, methanesulfonic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid; salts made with saccharin; alkali metal salts, such as sodium and potassium salts; alkaline earth metal salts, such as calcium and magnesium salts; and salts formed with organic or inorganic ligands, such as quaternary ammonium salts. In a preferred embodiment, the salt is neramexane mesylate (C₁₁H₂₃N·CH₄O₃S, MW 265.42). The term "salts" can also include addition salts of free acids or free bases. All of these salts (or other similar salts) may be prepared by conventional means. All such salts are acceptable provided that they are non-toxic and do not substantially interfere with the desired pharmacological activity.

The present invention further includes all individual enantiomers, diastereomers, racemates, and other isomers of neramexane. The invention also includes all polymorphs and hydrates of neramexane. Such isomers, polymorphs, and hydrates may be prepared by methods known in the art, such as by crystallization from different solvents, by regiospecific and/or enantioselective synthesis and resolution, based on the disclosure provided herein.

Neramexane, and thus the compositions of the present invention, are useful for the prevention and/or treatment of a number of diseases and conditions affecting the central nervous system (CNS), including dementia, Alzheimer's disease, Parkinson's disease, AIDS-related dementia, neuropathic pain, epilepsy, and depression. Other diseases in which the compositions are beneficial include glaucoma, hepatic encephalopathy, multiple sclerosis, stroke, dyskinesia, malaria, and viral infections such as hepatitis C. In a preferred embodiment, the compositions are used for the management of Alzheimer's disease and other types of dementia.

Optionally, the composition may further comprise another active ingredient which is preferably not a neramexane. As used herein, an active ingredient is a pharmaceutically acceptable compound or mixture of compounds useful for the diagnosis, prevention, or treatment of a symptom, disease, or condition. The terms "active compound", "active ingredient", "drug", and "drug substance" may be used interchangeably.

In one embodiment, this other active ingredient is effective in the management of CNS-related conditions or diseases. These conditions may be the same as the one which is to be treated by the neramexane, such as Alzheimer's disease or other types of dementia; or it may be useful for the management of other symptoms and conditions which are frequently present in patients suffering from Alzheimer's disease or dementia. Alternatively, the other active ingredient may be suitable to treat common side effects of NMDA receptor antagonists.

For example, a patient suffering from Alzheimer's disease may also have to be treated with an antidepressant, antipsychotic, anti-Parkinson agent, or sedative. Other drug classes from which the other active ingredient may be selected include acetylcholinesterase inhibitors such as donepezil, galantamine, rivastigmine, or tacrine.

### FORMULATION

As used herein, aqueous liquid pharmaceutical compositions by definition include liquid solutions and dispersions, such as emulsions, and semi-solid forms such as suspensions, creams, ointments and gels. More preferably, the composition of the invention is a liquid solution. An aqueous liquid composition is a liquid preparation whose major liquid component is water. Optionally, the aqueous liquid composition may further comprise other liquid components, such as pharmaceutically acceptable organic solvents. Examples of such other liquid components are ethanol, glycerol, propylene glycol, and polyethylene glycol. In a preferred embodiment, water is the only liquid component of the composition of the invention.

Such pharmaceutical compositions comprise a therapeutically effective amount of one or more of the foregoing active ingredients dissolved in a pharmaceutically acceptable solvent, optionally a taste masking agent and optionally an antimicrobial and/or preservative agent. The taste masking component may be a sweetener. The taste masking component may further comprise a flavorant. A solubilizer may also be included to keep ingredients with a tendency to crystallize from doing so. Additional optional excipients that may be added include solvents, flavorings, carriers, stabilizing agents, binders, colorants, antioxidants, and buffers (all pharmaceutically acceptable).

The active ingredient is Neramexane and its optical isomers, diastereomers, polymorphs, enantiomers, hydrates and pharmaceutically acceptable salts, e.g., HCl or mesylate. The active ingredient may be present in amounts ranging broadly from about 0.05 to about 5 %w/v, particularly ranging from about 0.1 to about 2 % w/v based on the total volume of the solution. In another embodiment, the active ingredient is present in an amount of about 0.2 % w/v (2mg /mL). In another embodiment, the active ingredient is present in about 0.5 % w/v (5 mg/mL). In yet another embodiment, the active ingredient is present in about 1.0 % w/v (10 mg/mL). In another embodiment, the active ingredient is present in about 2.0 % w/v (20 mg/mL).

According to one of the embodiments, the composition comprises neramexane, or a salt of neramexane, optionally neramexane mesylate.

While the antimicrobial effectiveness may somewhat differ between neramexane and its respective salts, it has been observed that in general, concentrations of less than about 1 mg/mL are not as effective in preserving liquid aqueous formulations. Marked antimicrobial activity is typical at a concentration of about 1-2 mg/mL, and becomes further pronounced at concentrations of about 5 mg/mL.

In a composition wherein neramexane mesylate is selected as active ingredient, the drug concentration in the composition may be in the range from about 2 mg/mL to about 100 mg/mL. A concentration in the range from about 5 mg/mL to about 10 mg/mL provides both effective preservation and convenient dosing.

According to the invention, aqueous compositions comprising neramexane can be formulated without preservatives, and preferably also without excipients having antimicrobial activity. Surprisingly, neramexane has been found to exhibit significant antimicrobial activity at concentrations which are useful for pharmaceutical formulation purposes.

In one embodiment, the composition of the invention is free of preservatives. In this context, free of preservatives means that preservatives are not detectable in the composition, or only in concentrations which are generally considered irrelevant with regard to any preservation effects.

According to the present invention, preservatives are defined as excipients having substantial antimicrobial activity. Substantial antimicrobial activity means that the activity is sufficient to ensure the microbiological quality of a product at a low concentration, such as at concentrations of 2-3 % (w/v) or less, or at a concentration at which the preservative is physiologically acceptable in relation to the volume in which the product is administered.

In another embodiment, the composition comprises at least one preservative, but at a concentration which is insufficient to effectively preserve the corresponding placebo composition. As used herein, a placebo composition is a formulation which is substantially free of active ingredients. A corresponding placebo composition is defined as a drug-free composition whose properties and other ingredients are largely the same as those of the drug-containing reference composition.

Whether a composition is effectively preserved is determined with the test for preservative efficacy (USP <51>), wherein five challenge organisms are tested at defined time intervals, depending on the product category. Conducted in appropriate series, such testing may also be performed in order to determine the minimally effective concentration of a specific preservative for a given composition, such as a drug-free composition corresponding to a composition according to the invention. For example, it may be found that in order to effectively preserve a particular placebo composition with sorbic acid, the preservative must be present at a concentration of at least about 0.1 % (w/v). In this case, the reference composition which comprises the neramexane, if it is a composition of the invention, could contain sorbic acid at a substantially lower concentration, such as about 0.05 % (w/v) or less. In another embodiment, the concentration of the preservative is selected to be not more than about a fifth, and more preferably not more than about a tenth, of the concentration needed to effectively preserve a corresponding placebo composition.

Since the microbiological quality of the composition of the invention is ensured fully or in part by the active compound itself, the composition is potentially superior to conventional formulations in terms of tolerability and safety.

Representative preservatives in such pharmaceutical preparations may include methyl paraben, ethyl paraben, propyl paraben, benzoic acid, sodium benzoate, propionic acid, sodium propionate, sorbic acid, potassium sorbate, bronopol, chlorbutol, benzyl alcohol, phenol, thiomersal, cetylpyridinium and benzalkonium chloride, to mention only a few. The concentrations and conditions at which preservatives effectively prevent microbial growth may differ widely and are understood in the art. For example, methyl paraben is typically effective at a concentration of about 0.1 to about 0.2 % (w/v), whereas propyl paraben can be incorporated at a concentration of only about 0.02 to about 0.03 % (w/v) to produce the same preservative effect. The pH of the liquid to be preserved may also play an important role. For example, sorbic acid, potassium sorbate, benzoic acid, and sodium benzoate are much more effective at an acidic pH than in neutral environments.

In one embodiment, a combination of methylparaben:propylparaben is used in a ratio of 10:1. In certain embodiments, methyl paraben is present in amounts ranging broadly from about 0.05% to about 2.0% w/v, optionally from about 0.1 to about 1.0 % w/v, more particluarly in an amount of about 0.1% w/v. In certain embodiments, propylparaben is present in amounts ranging broadly from about 0.005% to about 0.02%w/v, optionally from about 0.005 to about 0.01 %w/v, more particularly in an amount of about 0.01% w/v.

Other excipients which are usually not classified as preservatives may possess antimicrobial activity at somewhat higher concentrations such as above 15 or 20 % (v/v), for example ethanol. Nevertheless, in formulations which contain substantial amounts of any of these excipients, the use of other preservatives may not be necessary.

In a composition designed for oral administration, it is recommended to incorporate one or more excipients which improve the taste of the formulation. This is particularly true for neramexane mesylate. For example, at least one sweetener may be incorporated. Furthermore, one or more excipients selected from the group of flavors, flavor enhancers, and taste masking agents may be added.

Sweeteners, as used herein, are natural or synthetic compounds which have a sweet taste and are physiologically acceptable. Prominent examples of natural sweeteners include common sugars and sugar alcohols such as sucrose, glucose, fructose, maltose, maltitol, xylitol, lactitol, mannitol, and sorbitol. Preferably, a sugar alcohol is used to improve the flavor of the composition of the invention, in particular sorbitol. A useful concentration range for sorbitol or other sugars and sugar alcohols is from about 5 % (w/v) to about 40 % (w/v), and more preferably around 10-30 % (w/v).

In another embodiment, an artificial sweetener is incorporated in the composition in addition to, or instead of, a natural sweetener. Useful artificial sweeteners include saccharin-sodium, saccharin, sodium cyclamate, acesulfame K, neohesperidine dihydrochalcone, and aspartame, as well as any other sweeteners whose safety in human use is established. Appropriate concentrations depend on the individual sweetener which is selected.

The oral pharmaceutical composition of the invention may be in the form of a "tastemasked" or "taste-neutral" form. As certain forms of the active ingredient may have bitter taste, the solutions may contain any pharmaceutically acceptable sweeteners and/or flavoring agent. Flavorings may be used as necessary, including for example Natural peppermint #104, artificial cherry #10641, artificial grape #255, orange N&A 583K or artificial grape bubble gum # 998. These are commercially available, e.g., from Virginia Dare (Brooklyn, NY). In one embodiment, flavorings are added in a concentration ranging from about 0.04 to about 5 % w/v, preferably from about 0.05 to about 2.0% w/v, most preferred in an amount of about 0.05% w/v to the final formulation. In another embodiment, a flavoring concentration of about 0.5% is the most preferred amount. In another embodiment, flavoring concentration of about 1% w/v to the final formulation is the most preferred amount.

The flavor enhancers useful for practicing the invention may typically be sweetness enhancers, such as inositol. For example, the taste masking agent may be selected from the group of physiologically acceptable natural or synthetic gums.

For reproducible product quality and reliable stability, it is further preferred that the composition is adjusted to a specific pH by incorporating one or more appropriate excipients selected from the group consisting of physiologically acceptable acids, bases, and acidic and alkaline salts. For example, the combination of citric acid and sodium citrate may be used for buffering the pH of the composition at a value selected in the range from about pH 5 to about pH 8. More preferably, the pH is adjusted to a value from about pH 5.5 to about pH 7. One or more buffers are used as necessary, but preferably in amounts ranging from about 1 mg/ml to about 10 mg/ml. For example, citric acid may be present in an amount ranging broadly from about 0.1 to about 0.4 %w/v, preferably in an amount ranging from about 0.15 to 0.23%w/v, most preferably in an amount of about 0.19%w/v. Sodium citrate may be present in an amount ranging broadly from about 0.75 to about 2 %w/v, preferably from about 0.84 to about 1.0%w/v, most preferred in an amount of about 0.88 %w/v.

Further excipients which are routinely used in pharmaceutical formulations may be incorporated as may seem appropriate to adjust the composition to the specific requirements of a particular drug candidate, or to a specific use or target population. Examples of potentially suitable excipients are thickeners such as soluble gums including carrageenan, alginate, xanthan, and soluble cellulose esters; coloring agents; stabilizers, such as antioxidants, or crystallization inhibitors, such as glycerol, propylene glycol, or polyvinylpyrrolidone.

The formulation of the present invention also contains solubilizers that serve to enhance solubility of the parabens, sorbitol, and flavoring agents, and thus serve to reduce or eliminate closure locking. The amount of solubilizer should be carefully adjusted, however, to prevent or reduce the chance of leakage of the composition from the container through the closure such as might be experienced on transportation or upon tipping during storage or use. Appropriate solubilizers include propylene glycol, polyethylene glycol, and glycerin. Preferably, glycerin is used. the preferred amounts used will be specific for each formulation. Solubilizers may be used in amounts generally ranging from about 1 mg/ml to about 200 mg/ml. For example, propylene glycol, when used, is present in an amount ranging broadly from about 1 to about 4%w/v, preferably from about 2 to about 3%w/v, most preferred in an amount of about 2.5%. Glycerol, when used, is present in an amount ranging broadly from about 8 to about 12%w/v, preferably from about 9 to about 11 %w/v, most preferably in an amount of about 10%w/v. The use of a solubilizer may affect the pH of the solution. In that case, pH should be adjusted to be in the range of about 4 to about 7, preferably in the range of about 4.5 to about 6.5, most preferably about 5.5.

In a preferred embodiment, the vehicle for the formulation may be purified water or mixtures of water and ethanol. Preferably, solvents are used QS. In certain embodiments, the oral solution of the Neramexane Mesylate is in four strengths, 2 mg/ml, 5 mg/ml, 10 mg/ml and 20 mg/ml. Any appropriate bottle known in the art may be used for packaging. Any suitable screw cap closure can be used, preferably, a child resistant screw cap closure with a laminated seal activated by heat. Preferably, the packaging for the oral solutions includes six configurations, 120 ml, 360 ml, and 480 ml amber PET oblong shaped bottles with a child resistant heat seal cap or 20 ml, 50 ml, and 100 ml round brown glass bottles with a dropper and closure cap.

In addition to the high microbiological stability of the composition which has been discussed above in detail, it is another advantage of the invention that the composition can be manufactured easily and economically using standard equipment. Neramexane is usually available in salt forms which are water soluble, such as neramexane mesylate. The same is true for many other preferred excipients mentioned herein, so that the composition can usually be prepared from the active compound, the solid excipients and purified water simply by mixing the components under some agitation. In most cases, no heating or homogenization will be necessary. In other cases, depending on the specific selection of excipients, some heating may be recommended.

In a composition designed for parenteral use, the excipients, and in particular the water, should be sterile (e.g. water for injection) or have a low level of microbial contamination (bio-burden). The manufacturing process must be designed, validated, and conducted to ensure the high quality level which is generally required for parenteral products, and to comply with current GMP standards. Usually, the process will include a step of sterilization of the product within its final container. The standards and the regulatory guidances relating to the manufacture of sterile products are well known to persons skilled in this art.

According to one of the embodiments, the composition is designated for oral administration. In this case, the composition is preferably filled into containers which hold a plurality of doses. Appropriate containers will hold a volume in the range from about 5 mL or 5 g to about 1,000 mL or 1,000 g, and more preferably from about 10 mL (or g) to about 500 mL (or g). The volume is selected in consideration of the strength of the specific formulation and the time period for which the product is to be used. For example, a container may be selected to accommodate the medication needed for several days, weeks, or months. In one of the preferred embodiments, the container is selected to hold sufficient medication for at least about 4 weeks. In another embodiment, the container is selected to hold about 50 mL (or g), about 100 mL (or g), about 200 mL (or g), about 250 mL (or g), or about 500 mL (or g).

Appropriate containers may be of glass or a suitable plastic material, such as polypropylene or polyethylene, and will usually have a container closure system which is reclosable. Optionally, the closure system is child-proof.

The container may further comprise a means for measuring and /or dispensing defined doses of the composition. A conventional measuring means is, for example, a dropper, i.e. a glass tube fitted with a rubber bulb which is integrated in the closure and removed when opening the container. Alternatively, a non-removable dropper may be integrated in the bottle neck.

In another embodiment, the container closure system, comprises a dosing cup that provides markings indicating the amount of liquid to be taken for the most common doses. For example, the markings may range from about 0.5 mL to about 10 mL, and more preferably from about 1 mL to about 5 mL, or instead of volumes they may indicate the dose in grams of formulation, or in mg of drug substance. The measuring cup may be part of the container closure system, or it may be provided as a separate device within the secondary package in which the container is presented.

### DOSAGE AND ADMINISTRATION

A representative aqueous liquid composition of the instant invention includes an effective amount of neramexane to provide from about 1 mg/day to about 100 mg/day, preferably from about 5 mg/day to about 80 mg/day most preferably from about 10 to about 60 mg/day. Smaller initial doses can be used, eventually increased to at least about 10 mg within the aforementioned ranges. The drug may be administered once a day, BID or more often.

The formulated solution of the present invention is preferably a sugar-free, alcohol-free, palatable liquid solution stable enough for long-term use.

### DEFINITIONS

A "therapeutically effective amount" of a drug is an amount effective to demonstrate a desired activity of the drug. According to the instant invention, in one embodiment a therapeutically effective amount of memantine is an amount effective to treat CNS disorders, *i.e*., dementia or neuropathic pain.

As used herein, the term "pharmaceutically acceptable" refers to a biologically or pharmacologically compatible for *in vivo* use, and preferably means approved by a regulatory agency of the Federal or a state government or listed in the U.S. Pharmacopeia or other generally recognized pharmacopeia for use in animals, and more particularly in humans.

The term "about" or "approximately" means within an acceptable error range for the particular value as determined by one of ordinary skill in the art, which will depend in part on how the value is measured or determined, *i.e*., the limitations of the measurement system. For example, "about" can mean within 1 or more than 1 standard deviations, per the practice in the art. Alternatively, "about" can mean a range of up to 20%, preferably up to 10%, more preferably up to 5%, and more preferably still up to 1 % of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

The following examples are presented to further illustrate the invention. However, they are not to be construed as to limit the scope thereof.

### EXAMPLES .

### Example 1 (comparative example)

Memantine hydrochloride (5.0 g) was dissolved in purified water (Ph. Eur., 10 L) to prepare a solution of 0.5 mg/mL. No preservative was added. The solution was filled into 10 mL glass bottles with screw closures. Samples were drawn for conducting the test for preservative efficacy according to Ph. Eur. The test involved a challenge of the samples with the following species:
- Escherichia coli (A)
- Pseudomonas aeruginosa (B)
- Staphylococcus aureus (C)
- Candida albicans (D)
- Aspergillus niger (E)

The initial contamination and its changes in the subsequent 28 d were quantified as colony-forming units per mL (CFU/mL) as shown in table 1.

**Table 1 - Antimicrobial Test Results for Memantine HCl Solution (0.5 mg/mL)**

| Time | A | B | C | D | E |
|---|---|---|---|---|---|
| 0 | 270,000 | 350,000 | 250,000 | 260,000 | 200,000 |
| 6 h | 600 | <100 | 3,000 | 40,000 | 220,000 |
| 24 h | 300 | <100 | <100 | 900 | 220,000 |
| 7 d | 0 | 0 | 0 | 0 | 200,000 |
| 14 d | 0 | 0 | 0 | 0 | 160,000 |
| 21 d | 0 | 0 | 0 | 0 | 180,000 |
| 28 d | 0 | 0 | 0 | 0 | 180,000 |

The results indicate that the tested solution is not microbiologically stable as it is not effectively preserved against mold contamination.

### Example 2 (comparative example)

Neramexane mesylate (5.0 g) was dissolved in purified water (Ph. Eur., 10 L) to prepare a solution of 0.5 mg/mL. No preservative was added. The solution was filled into 10 mL glass bottles with screw closures. Samples were drawn and tested as described in example 1. The results of the microbial challenge test are given as CFU/mL in table 2.

**Table 2 - Antimicrobial Test Results for Neramexane mesylate Solution (0.5 mg/mL)**

| Time | A | B | C | D | E |
|---|---|---|---|---|---|
| 0 | 270,000 | 350,000 | 250,000 | 260,000 | 200,000 |
| 6 h | 1,500 | <100 | 300 | 55,000 | 160,000 |
| 24 h | <100 | 0 | 200 | 36,000 | 160,000 |
| 7 d | 0 | 0 | <100 | 20,000 | 180,000 |
| 14 d | 0 | 0 | 0 | 318,000 | 180,000 |
| 21 d | 0 | 0 | 0 | 409,000 | 180,000 |
| 28 d | 0 | 0 | 0 | 840,000 | 200,000 |

Again, the results indicate that the tested solution is not microbiologically stable. In this case, it is not effectively preserved against yeast and mold contamination.

### Example 3 Memantine HCl aqueous solution(outside the scope of the invention)

Preservative-free aqueous solutions of memantine hydrochloride with concentrations of 5 mg/mL, 10 mg/mL, 20 mg/mL, and 40 mg/mL were prepared using purified water (Ph. Eur.). No preservatives were added. Samples were drawn and tested as described in example 1. The results are shown as CFU/mL in table 3 (for 5 mg/mL), table 4 (for 10 mg/mL), table 5 (for 20 mg/mL), and table 6 (for 40 mg/mL).

**Table 3 - Antimicrobial Test Results for Memantine HCl Solution (5 mg/mL)**

| Time | A | B | C | D | E |
|---|---|---|---|---|---|
| 0 | 270,000 | 350,000 | 250,000 | 260,000 | 200,000 |
| 6 h | 400 | 0 | 0 | <100 | 1,200 |
| 24 h | 0 | 0 | 0 | 0 | 200 |
| 7 d | 0 | 0 | 0 | 0 | 0 |
| 14 d | 0 | 0 | 0 | 0 | 0 |
| 21 d | 0 | 0 | 0 | 0 | 0 |
| 28 d | 0 | 0 | 0 | 0 | 0 |

**Table 4 - Antimicrobial Test Results for Memantine HCl Solution (10 mg/mL)**

| Time | A | B | C | D | E |
|---|---|---|---|---|---|
| 0 | 270,000 | 260,000 | 210,000 | 280,000 | 240,000 |
| 14 d | 0 | 0 | 0 | 0 | 1,500 |
| 28 d | 0 | 0 | 0 | 0 | <100 |

**Table 5 - Antimicrobial Test Results for Memantine HCl Solution (20 mg/mL)**

| Time | A | B | C | D | E |
|---|---|---|---|---|---|
| 0 | 270,000 | 260,000 | 210,000 | 280,000 | 240,000 |
| 6 h | 0 | 0 | 0 | <100 | 64,000 |
| 24 h | 0 | 0 | 0 | 0 | 20,000 |
| 7 d | 0 | 0 | 0 | 0 | 1,200 |
| 14 d | 0 | 0 | 0 | 0 | 200 |
| 21 d | 0 | 0 | 0 | 0 | 100 |
| 28 d | 0 | 0 | 0 | 0 | 0 |

**Table 6 - Antimicrobial Test Results for Memantine HCl Solution (40 mg/mL)**

| Time | A | B | C | D | E |
|---|---|---|---|---|---|
| 0 | 270,000 | 260,000 | 210,000 | 280,000 | 240,000 |
| 6 h | 0 | 0 | 0 | 0 | 20,000 |
| 24 h | 0 | 0 | 0 | 0 | 1,400 |
| 7 d | 0 | 0 | 0 | 0 | 200 |
| 14 d | 0 | 0 | 0 | 0 | 100 |
| 21 d | 0 | 0 | 0 | 0 | 0 |
| 28 d | 0 | 0 | 0 | 0 | 0 |

The results demonstrate that all tested solutions were microbiologically stable and effectively preserved against microbial contamination.

### Example 4 Neramexane mesylate aqueous solution

Preservative-free aqueous solutions of neramexane mesylate with concentrations of 5 mg/mL, 10 mg/mL, 50 mg/mL, and 250 mg/mL were prepared using purified water (Ph. Eur.). No preservatives were added. Samples were drawn and tested as described in example 1. The results are shown as CFU/mL in table 7 (for 5 mg/mL), table 8 (for 10 mg/mL), table 9 (for 50 mg/mL), and table 10 (for 250 mg/mL).

**Table 7 - Antimicrobial Test Results for Neramexane mesylate Solution (5 mg/mL)**

| Time | A | B | C | D | E |
|---|---|---|---|---|---|
| 0 | 270,000 | 350,000 | 250,000 | 260,000 | 200,000 |
| 6 h | 0 | 0 | 0 | 0 | 6,000 |
| 24 h | 0 | 0 | 0 | 0 | 2,800 |
| 7 d | 0 | 0 | 0 | 0 | 0 |
| 14 d | 0 | 0 | 0 | 0 | 0 |
| 21 d | 0 | 0 | 0 | 0 | 0 |
| 28 d | 0 | 0 | 0 | 0 | 0 |

**Table 8 - Antimicrobial Test Results for Neramexane mesylate Solution (10 mg/mL)**

| Time | A | B | C | D | E |
|---|---|---|---|---|---|
| 0 | 270,000 | 240,000 | 280,000 | 330,000 | 200,000 |
| 6 h | 0 | 0 | 0 | 0 | 400 |
| 24 h | 0 | 0 | 0 | 0 | 300 |
| 7 d | 0 | 0 | 0 | 0 | 0 |
| 14 d | 0 | 0 | 0 | 0 | 0 |
| 21 d | 0 | 0 | 0 | 0 | 0 |
| 28 d | 0 | 0 | 0 | 0 | 0 |

**Table 9 - Antimicrobial Test Results for Neramexane mesylate Solution (50 mg/mL)**

| Time | A | B | C | D | E |
|---|---|---|---|---|---|
| 0 | 220,000 | 300,000 | 260,000 | 230,000 | 270,000 |
| 6 h | 0 | 0 | 0 | 0 | 18,000 |
| 24 h | 0 | 0 | 0 | 0 | 400 |
| 7 d | 0 | 0 | 0 | 0 | <100 |
| 14 d | 0 | 0 | 0 | 0 | 0 |
| 21 d | 0 | 0 | 0 | 0 | 0 |
| 28 d | 0 | 0 | 0 | 0 | 0 |

**Table 10 - Antimicrobial Test Results for Neramexane mesylate Solution (250 mg/mL)**

| Time | A | B | C | D | E |
|---|---|---|---|---|---|
| 0 | 220,000 | 300,000 | 260,000 | 230,000 | 270,000 |
| 6 h | 0 | 0 | 0 | 0 | 800 |
| 24 h | 0 | 0 | 0 | 0 | 100 |
| 7 d | 0 | 0 | 0 | 0 | 0 |
| 14 d | 0 | 0 | 0 | 0 | 0 |
| 21 d | 0 | 0 | 0 | 0 | 0 |
| 28 d | 0 | 0 | 0 | 0 | 0 |

The results demonstrate that all tested solutions were microbiologically stable and effectively preserved against microbial contamination.

### Example 5: Memantine Oral Solution (outside scope of the invention)

This Example demonstrates the process of making a memantine oral solution. The following ingredients in Table 11 were combined according to the process described below.

**Table 11. Composition make-up**

| **Strength** | **2 mg/ml** | **4 mg/ml** |
|---|---|---|
| **Ingredients** | **% w/v (mg/ml in parentheses)** | **% w/v (mg/ml in parentheses)** |
| Memantine HCl | 0.20 (2) | 0.40 (4.0) |
| Sorbitol solution, USP 70% | 30.00 (300) | 30.00 (300) |
| Methyl paraben, NF | 0.1 (1.00) | 0.1 (1.00) |
| Propyl Paraben, NF | 0.01 (0.10) | 0.01 (0.10) |
| Propylene Glycol, USP | 2.50 (25) | 2.50 (25) |
| Glycerin, USP | 10.00 (100) | 10.00 (100) |
| Natural Peppermint Flavor #104 | 0.05 (0.50) | 0.05 (0.50) |
| Citric Acid, USP | 0.19 (1.92) | 0.19 (1.92) |
| Sodium Citrate, USP | 0.88 (8.82) | 0.88 (8.82) |
| Purified Water, USP | QS | QS |

For each composition strength, purified water was heated to 85°C, and then cooled to 20-30°C in a 1000 gallon tank. In a separate batch tank, sorbitol 70% was mixed with purified water, QS to approximately 2500L. To the sorbitol-water solution, citric acid and sodium citrate were added and mixed. Glycerin was then added, followed by memantine hydrochloride. In a separate 55 gallon tank, a sub-solution of propylene glycol, methyl paraben, propyl paraben, and natural peppermint flavor #104 was mixed. The sub-solution was then added to the batch tank, which was subsequently QS to 3785L with the purified water from the 1000 gallon tank. The final solution was cooled below 30°C, then to 20-25°C. The solution was filtered, filled into bottles and then capped.

The formulations were tested for taste. The taste evaluation study was performed with four healthy subjects. Since memantine has a characteristics bitter taste, the subjects were asked to rate the formulation. Each subject took a tea spoon (about 5 mL) of solution and rated the product as follows:
Good: No bitter taste and solution taste is acceptable
Poor: Bitter taste
Poor: The solution taste is unacceptable.

Taste of both the 4 mg/ml and 2 mg/ml formulation was good and devoid of bitter after taste.

### Example 6: Stability of Memantine Oral Solution (outside scope of the invention)

In the present Example, the stability of the solutions made in Example 5 was tested for percent of memantine, methyl paraben, propyl paraben, degradation and pH. The stability study of the 4 mg/mL scale up batch was initiated at 40°C/75% relative humidity using 120 cc oval amber bottles, 24/400 CRC with heat seal liner.

The stability of the solutions were determined using a HPLC method, using an HPLC system with autosampler, column-temperature-controller, UV detector, and HPLC syringe pump for postcolumn reagents. The eluted drug, which is derivatized with o-Phthaldehyde after HPLC separation is detected and quantitated using UV detection at 340 nm. The column RP8 (Waters Xterra) is packed with octylesilane chemically bonded with embedded polar reversed-phased ligand utilizing hybrid particle technology. The packing material are porous spherical with pore size of 125 A with a size of 3.5 µm. The HPLC conditions were as follows:

| | |
|---|---|
| Column: | Waters Xterra, RP8 HPLC, 3.0 x 100 mm,3.5 µm or equivalent |
| Column Temperature: | 50°C |
| Flow Rate: | 0.75 mL/min |
| Injection Volume: | 20 µL |
| UV Detector: | 340 nm |
| Run Time: | 5 minutes |
| Injector Washing Solution: | Methanol:Water, [50:50 (v:v)] (recommended) |
| Mobile Phase: | 0.1 % TFA and 20% (v/v) Acetonitrile in Water |

The post-column conditions were as follows:

| | |
|---|---|
| Reagent: | 5 g/L O-Phthaldehyde (OPA) and 5 mUL 3-Marcaotoproprionic acid (MPA) in 1:9 (v/v) Acetonitrile:0.3 M pH 10.4 Borate Buffer |
| Flow Rate: | 0.25 mL/min |
| Reagent Pre-Heating Coil: | 1,575 µL (Alltech P/N: 35896) |
| Reactor Coil: | 700 µL (Alltech P/N: 35886) |
| Reactor Temperature: | 50 °C |

The data have been summarized below in Table 12.

**Table 12. Stability Data**

| Conditions | Assay, Memantine HCl | Assay, Methylparaben | Assay, Propylparaben | Degradation products | pH |
|---|---|---|---|---|---|
| Temperature / Relative Humidity X Months | % | % | % | % | |
| Initial | 99.3 | 98.4 | 98.4 | ND* | 5.4 |
| 40°C/75%RHx1M | 100.6 | 100.7 | 100.3 | ND | 5.5 |
| 40°C/75%RHx3M | 102.1 | 98.2 | 98.3 | ND | 5.4 |
| 25 °C/60% RH x3M | 103.4 | 100.5 | 101.7 | ND | 5.4 |
| 40°C/75%RHx6M | 102.3 | 97.8 | 98.2 | ND | 5.5 |
| 25°C/60%RH x 6M | 101.2 | 100.4 | 100.3 | ND | 5.5 |

| | | | | | |
|---|---|---|---|---|---|
| *Not detected | | | | | |

The formulation was still found to be stable after 6 months. Results of assay, pH, and preservative show that values are between 90 to 110% showing excellent stability of the solution at accelerated 40°C/75% relative humidity conditions for six months. In addition, degradation products are not detected.

Although the scaled-up batch showed good results, similar measurements were conducted as an in-use stability study where bottles were handled to mimic in-use conditions. 8 bottles of memantine oral solution, 4mg/ml were stored at room temperature without humidity control. Bottles were opened daily (5 days/week) for 5 minutes to stimulate conditions during normal use. After 5 minutes, the bottles were closed. The samples were analyzed after 2, 4, and 6 weeks to determine assay of antimicrobials, parabens, degradation products, pH and preservative effectiveness. Results are shown in Table 13 below.

**Table 13. In use stability data**

| Test | Initial | RT/2 wks | RT/ 4 wks | RT/ 6 wks |
|---|---|---|---|---|
| Assay of memantine HCl | 100.6 | 99.8 | 99.2 | 99.6 |
| Assay of methyl paraben | 103.2 | 103.8 | 104.1 | 103.9 |
| Assay of propyl paraben | 102.7 | 99.3 | 100.1 | 101.6 |
| Degradation products | - | Not detected | Not detected | Not detected |
| pH | 5.42 | 5.46 | 5.46 | 5.46 |

Antimicrobial Effectiveness Testing was conducted to demonstrate that the formulations contained antimicrobial preservatives to protect the formulation from microbiological growth or from microorganisms that were introduced inadvertently or subsequent to manufacturing process. The testing was performed in accordance with the USP <51> using the culture conditions for inoculum specified in the test conditions.

The Antimicrobial Effectiveness Testing (referred to later in the text as APE or antimicrobial effectiveness) is performed as described in USP 26, The United States Pharmacopeial Convention, Inc. (Rockville, MD, 2002; pp. 2002 - 2004). The test is conducted in five sterile, capped bacteriological containers into which a sufficient volume of product has been transferred. Test organisms include *Candida albicans* (ATCC No. 10231), *Aspergillus niger* (ATCC No. 16404), *Escherichia coli* (ATCC No. 8739), *Pseudomonas aeruginosa* (ATCC No. 9027), *Staphylococcus aureus* (ATCC No. 6538). Each container is inoculated with one of the prepared and standardized inoculum, and mix. The concentration of test microorganisms that is added to the product are such that the final concentration of the test preparation after inoculation is between 1 x 10⁵ and 1 x 10⁶ cfu per mL of the product. The inoculated containers are incubated at 22.5 ± 2.5° C, and sampled at the appropriate intervals specified in the monograph. The number of cfu present in each test preparation is determined by the plate-count procedure, specified in the monograph, for the applicable intervals. Using the calculated concentrations of cfu per mL present at the start of the test, the change in log₁₀ values of the concentration of cfu per mL for each microorganism is calculated at the applicable test intervals, and the changes in terms of log reductions is expressed. Results are evaluated in accordance with the Product Category for Oral Products made with aqueous bases or vehicle.

The Antimicrobial test results for Pseudomonas aeruginosa (ATCC 9027), Escherichia coli (ATCC 8739), Staphylococcus aureus (ATCC 6538), Candida albicans (ATCC 10231), and Aspergillus niger (ATCC 16404) are listed in Table 14 below

**Table 14. Antimicrobial Test Results for the In-use stability samples.**

| Inoculum: | Pseudomonas aeruginosa | | Escherichia coli | | Staphylococcus aureus | | Candida albicans | | Aspergillus niger | |
|---|---|---|---|---|---|---|---|---|---|---|
| ATCC No. | AT CC 9027 | | ATC C 8739 | | ATCC 6538 | | ATCC 10231 | | ATCC 16404 | |
| | CFU /mL | Log Reduction | CFU/ mL | Log Reduction | CFU/mL | Log Reductio n | CFU/mL | Log Redu ction | CFU/m L | Log Reducti on |
| Initial | | | | | | | | | | |
| Time Initial | 1.8 X 10⁵ | | 4.5 X 10⁵ | | 8.0X10⁵ | | 2.3 X 10⁵ | | 1.5 X 10⁵ | |
| 14 Days | <10 | 5.3 | <10 | 5.7 | <10 | 5.9 | <10 | 5.4 | <10 | 5.2 |
| 28 Days | <10 | 5.3 | <10 | 5.7 | <10 | 5.9 | <10 | 5.4 | <10 | 5.2 |

| RT/2 Weeks | | | | | | | | | | |
|---|---|---|---|---|---|---|---|---|---|---|
| Time Initial | 1.8 X 10⁵ | | 4.5 X 10⁵ | | 8.0 X 10⁵ | | 2.3 X 10⁵ | | 1.5 X 10⁵ | |
| 14 Days | < 10 | 5.3 | <10 | 5.7 | <10 | 5.9 | <10 | 5.4 | <10 | 5.2 |
| 28 Days | < 10 | 5.3 | <10 | 5.7 | <10 | 5.9 | <10 | 5.4 | <10 | 5.2 |

| RT/4 Weeks | | | | | | | | | | |
|---|---|---|---|---|---|---|---|---|---|---|
| Time Initial | 1.8 X 10⁵ | | 4.5 X 10⁵ | | 8.0 X 10⁵ | | 2.3 X 10⁵ | | 1.5 X 10⁵ | |
| 14 Days | < 10 | 5.3 | <10 | 5.7 | <10 | 5.9 | <10 | 5.4 | <10 | 5.2 |
| 28 Days | < 10 | 5.3 | <10 | 5.7 | <10 | 5.9 | <10 | 5.4 | <10 | 5.2 |

| RT/6 Weeks | | | | | | | | | | |
|---|---|---|---|---|---|---|---|---|---|---|
| Time Initial | 1.8 X 10⁵ | | 4.5 X 10⁵ | | 8.0 X 10⁵ | | 2.3 X 10⁵ | | 1.5 X 10⁵ | |
| 14 Days | < 10 | 5.3 | <10 | 5.7 | <10 | 5.9 | <10 | 5.4 | <10 | 5.2 |
| 28 Days | < 10 | 5.3 | <10 | 5.7 | <10 | 5.9 | <10 | 5.4 | <10 | 5.2 |

The products met the USP <51> criteria tor antimicrobial effectiveness for all inoculums. The solution product was found to be stable for the entire study period based on the antimicrobial effectiveness testing.

### Example 7: Anti-cap Locking of Memantine Oral Solution (outside the scope of the invention)

In the present Example, the selection of the anti-caplocking agent is described.

As discussed earlier, Sorbitol 70% solution was added as a sweetener in the formulation. It has a tendency to crystallize on the threads of the bottle cap and interferes with cap removal, which results in caplocking, or with hermetic closure which may result in leakage. Glycerin reduces the tendency of sorbitol to crystallize.

To determine the optimal concentration of glycerin required to minimize cap-locking for a 10 mg/mL memantine formulation, a caplocking study was conducted on a memantine solution of 1 % w/v (10 mg/mL) and 0.2 %, 2 mg /mL. The compositions of 10 mg /mL solution and test results are shown in Tables 15 and 16. The compositions and test results for 0.2 % w/v (2 mg/mL) are shown in tables 17 and 18.

**Table 15. Formulations prepared using different concentrations of glycerin.**

| **Ingredients** | **Sample %w/w** | **A %w/w** | **B %w/w** | **C %w/w** | **D % w/w** | **E % w/w** |
|---|---|---|---|---|---|---|
| Memantine HCl | 1.0 | 1.0 | 1.0 | 1.0 | 1.0 | 1.0 |
| Sorbitol 70% | 30.0 | 30.0 | 30.0 | 30.0 | 30.0 | 30.0 |
| Methyl Paraben | 0.05 | 0.05 | 0.05 | 0.05 | 0.05 | 0.05 |
| Propyl Paraben | 0.005 | 0.005 | 0.005 | 0.005 | 0.005 | 0.005 |
| Propylene Glycol | 2.5 | 2.5 | 2.5 | 2.5 | 2.5 | 2.5 |
| Glycerin | 0 | 2.5 | 5.0 | 10.0 | 15.0 | 20.0 |
| Peppermint Flavor | 0.05 | 0.05 | 0.05 | 0.05 | 0.05 | 0.05 |
| Purified Water | 66.395 | 63.895 | 61.395 | 56.395 | 51.395 | 46.395 |

For each formulation, the necks of 25 bottles were dipped in the solution prior to applying caps. Application torque was measured using Kaps-All Electronic Torque Tester (Kaps-All Electronic Torque tester, Model EB550, Riverhead, NY). The torque testing was performed according to manufacturer's instructions. Five bottles were used to determine the initial removal torque and the remaining bottles were put in a 50°C oven. Bottles were withdrawn after 1, 2, 3 and 4 wks. Removal torque was measured at each time point. Formulations with 0, 2.5 and 5% glycerin showed high removal torque and a white film of crystallized sorbitol was also evident around the neck of the bottle. In formulations containing 10% glycerin no film was formed around the neck of the bottle and caps could be removed easily. However, at a concentration of 15% and above, caps were loose which could lead to leakage. Based on these tests, it was determined that 8% w/v to 12 % w/v glycerin formulations effectively prevented caplocking. Data are presented in Table 16.

**Table 16. Torque values for Table 15 formulations after 4 weeks at 50°C**

| 0% Glycerin | | | | | | |
|---|---|---|---|---|---|---|
| | #1 | #2 | #3 | #4 | #5 | #6 |
| Application Torque (lb-in) | 13.5 | 11.4 | 12.7 | 11.3 | 11.7 | 12.1 |
| Removal Torque (lb-in) | 20.3 | 21.2 | 20.2 | 17.8 | 21.5 | 20.2 |

| 2.5% Glycerin | | | | | | |
|---|---|---|---|---|---|---|
| Application Torque (lb-in) | 11.6 | 11.1 | 11.5 | 11.5 | 11.4 | 11.4 |
| Removal Torque (lb-in) | 16.8 | 17.4 | 19.5 | 20.3 | 19.2 | 18.6 |

| 5% Glycerin | | | | | | |
|---|---|---|---|---|---|---|
| Application Torque (lb-in) | 11.4 | 11.2 | 11.3 | 12.1 | 11.0 | 11.4 |
| Removal Torque (lb-in) | 20.8 | 21.2 | 17.5 | 23.4 | 21.1 | 20.8 |

| 10% Glycerin | | | | | | |
|---|---|---|---|---|---|---|
| Application Torque (lb-in) | 11.5 | 11.1 | 11.2 | 11.9 | 12.0 | 11.5 |
| Removal Torque (lb-in) | 7.9 | 10.9 | 12.9 | 14.4 | 12.6 | 11.7 |

| 15% Glycerin | | | | | | |
|---|---|---|---|---|---|---|
| Application Torque (lb-in) | 11.3 | 12 | 11.5 | 11.7 | 11.0 | 11.5 |
| Removal Torque (lb-in) | 4.0 | 7.6 | 3.3 | 10.7 | 4.0 | 5.9 |

| 20% Glycerin | | | | | | |
|---|---|---|---|---|---|---|
| Application Torque (lb-in) | 11.3 | 11.2 | 11.3 | 11.3 | 11.8 | 11.8 |
| Removal Torque (lb-in) | 5.2 | 6.4 | 7.6 | 3.4 | 10.6 | 6.6 |

The caplock study was repeated for the 2 mg/mL memantine formulation. Glycerin was added in concentration of 0, 5, 10 and 15% to the formulation and caplocking tendency was measured as above. The compositions tested are shown in Table 17. The data pertaining to torque values are shown in Table 18.

**Table 17. Formulations containing various amounts of glycerin and 2mg/mL active ingredient.**

| **Sample** | **A** | **B** | **C** | **D** |
|---|---|---|---|---|
| **Ingredients** | **% w/v** | **% w/v** | **% w/v** | **% w/v** |
| Memantine HCl | 0.2 | 0.2 | 0.2 | 0.2 |
| Sorbitol 70% | 30.0 | 30.0 | 30.0 | 30.0 |
| Methyl Paraben | 0.05 | 0.05 | 0.05 | 0.05 |
| Propyl Paraben | 0.005 | 0.005 | 0.005 | 0.005 |
| Propylene Glycol | 2.5 | 2.5 | 2.5 | 2.5 |
| Glycerin | 0 | 5.0 | 10.0 | 15.0 |
| Peppermint Flavor | 0.05 | 0.05 | 0.05 | 0.05 |
| Citric acid | 0.192 | 0.192 | 0.192 | 0.192 |
| Sodium citrate | 0.882 | 0.882 | 0.882 | 0.882 |
| Purified Water | QS | QS | QS | QS |

**Table 18. Torque values for Table 17 formulations after 4 weeks at 50°C**

| 0% Glycerin | | | | | | |
|---|---|---|---|---|---|---|
| | #1 | #2 | #3 | #4 | #5 | #6 |
| Application Torque (lb-in) | 11.2 | 11.0 | 11.1 | 11.4 | 11.0 | 11.1 |
| Removal Torque (lb-in) | 11.4 | 12.8 | 12.1 | 13.4 | 13.0 | 12.5 |

| 5% Glycerin | | | | | | |
|---|---|---|---|---|---|---|
| Application Torque (lb-in) | 11.6 | 11.1 | 1 11.2 | 12.2 | 11.0 | 11.4 |
| Removal Torque (lb-in) | 12.4 | 11.7 | 12.3 | 10.9 | 11.2 | 11.7 |

| 10% Glycerin | | | | | | |
|---|---|---|---|---|---|---|
| Application Torque (lb-in) | 12.9 | 11.3 | 11.6 | 11.5 | 12.0 | 11.9 |
| Removal Torque (lb-in) | 9.6 | 8.5 | 8.0 | 8.0 | 9.0 | 8.6 |

| 15% Glycerin | | | | | | |
|---|---|---|---|---|---|---|
| Application Torque (lb-in) | 11.4 | 11.2 | 11.8 | 11.4 | 11.5 | 11.5 |
| Removal Torque (lb-in) | 8.1 | 8.0 | 8.4 | 9.0 | 8.1 | 8.3 |

In formulations containing 10% glycerin, no film was formed around the neck of the bottle, and caps could be removed easily. However, at a concentration of 15%, caps were rendered free which could lead to leakage of contents and hence is not desirable.

Based on the data, it was determined that 10% w/v of glycerin for both 2 mg/mL and 4 mg/mL formulations are appropriate to prevent closure locking and leaking. Indeed, given the high solubility of the active ingredient based on the data, it is determined that 8% w/v to 12 % w/v of glycerin is appropriate to prevent closure locking and leaking of memantine solutions.

### Example 8: Neramexane Oral Solution

The present Example demonstrates the process of making a neramexane oral solution in 2, 5, 10, and 20 mg/mL strengths. The following ingredients in Table 19 were combined according to the process described below.

**Table 19. Composition make-up**

| **Strength** | **2 mg/mL** | **5 mg/ml** | **10 mg/ml** | **20 mg/ mL** |
|---|---|---|---|---|
| **Ingredients** | **%w/v** | **% w/v** | **% w/v** | **% w/v** |
| Neramexane Mesylate | 0.2 | 0.5 | 1.0 | 2.0 |
| Sorbitol Solution, USP, 70% | 30.0 | 30.0 | 30.0 | 30.0 |
| Methylparaben, NF | 0.10 | 0.10 | 0.10 | 0.10 |
| Propylparaben, NF | 0.01 | 0.01 | 0.01 | 0.01 |
| Propylene Glycol, USP | 2.5 | 2.5 | 2.5 | 2.5 |
| Glycerin, USP | 10.0 | 10.0 | 10.0 | 10.0 |
| Flavor, Natural Peppermint #104 | 0.5 | 0.5 | 0.5 | 0.5 |
| Citric Acid, USP, Anhydrous | 0.19 | 0.19 | 0.19 | 0.19 |
| Sodium Citrate, USP, Dihydrate | 0.88 | 0.88 | 0.88 | 0.88 |
| Purified Water, USP | QS | QS | QS | QS |

Preparation process for one-liter batch was as follows. Sorbitol 70% was mixed with purified water in a suitable stainless steel container. To the sorbitol-water solution, glycerin was added and mixed. Citric acid and sodium citrate were then added, followed by Neramexane Mesylate. All the above ingredients were mixed to dissolve in the batch tank. In a separate container, a sub-solution of propylene glycol, methylparaben, propylparaben, and natural peppermint flavor #104 was mixed. The sub-solution was then added to the batch tank, which was subsequently QS to desired volume with purified water. The solution was filled into bottles and then capped.

### Example 9: Stability of Neramexane Oral Solution

In the present Example, the stability of the 10 mg/mL solutions made in Example 8 were tested for percent of neramexane, methyl paraben, propyl paraben, and pH using the same procedures described in Example 6. The data are presented below in Table 20.

**Table 20. Stability Data**

| Conditions | Assay of Neramexane | Assay for Methylparaben | Assay for Propylparaben | pH |
|---|---|---|---|---|
| Initial | 101 | 100 | 99 | 5.42 |
| 40C/75%RH x1M | 104 | 100 | 97 | 5.43 |
| 40C/75%RH x3M | 103 | 100 | 98 | 5.42 |
| 40C/75%RH x3M | 99 | 98 | 95 | 5.43 |

The results show excellent accelerated stability of Neramexane solution. See Example 5/6

### Example 10: Antibacterial Effectiveness in Neramexane Oral Solution

In the present Example, the antimicrobial effectiveness was measured in the neramexane oral solutions. The same testing procedures outlined in Example 6 were used. Table 21 provides the test results for different strengths of neramexane mesylate oral solution (2, 5, and 10 mg/mL) without preservative.

**Table 21. Antimicrobial Test Results**

| **Inoculum:** | **Pseudomonas aeruginosa** | | **Escherichia coli** | | **Staphylococcus aureus** | | **Candida albicans** | | **Aspergillus niger** | |
|---|---|---|---|---|---|---|---|---|---|---|
| **ATCC No.** | **ATCC 9027** | | **ATCC 8739** | | **ATCC 6538** | | **ATCC 10231** | | **ATCC 16404** | |
| | CFU /ml | Log Reduction | CFU /ml | Log Reduction | CFU /ml | Log Reduction | CFU /ml | Log Reduction | CFU /ml | Log Reduction |
| **Strength: 2 mg/mL** | | | | | | | | | | |
| Time Initial | 1.5 X 10⁵ | | 1.2 X 10⁵ | | 8.0 X 10⁵ | | 3.6 X 10⁵ | | 4.0 X 10⁵ | |
| 14 Days | < 10 | 5.2 | < 10 | 5.1 | < 10 | 5.9 | < 10 | 5.6 | 6.2 X 10⁴ | 0.8 |
| 28 Days | < 10 | 5.2 | < 10 | 5.1 | < 10 | 5.9 | < 10 | 5.6 | 3.9 X 10³ | 1.0 |

| **Strength: 5 mg/mL** | | | | | | | | | | |
|---|---|---|---|---|---|---|---|---|---|---|
| Time Initial | 1.5X10⁵ | | X | | 8.0 X 10⁵ | | 3.6 X 10⁵ | | 4.0 X 10⁵ | |
| 14 Days | < 10 | 5.2 | < 10 | 5.1 | < 10 | 5.9 | < 10 | 5.6 | 3.1 X 10⁴ | 1.1 |
| 28 Days | < 10 | 5.2 | < 10 | 5.1 | < 10 | 5.9 | < 10 | 5.6 | 1.8 X 10³ | 2.3 |

| **Strength: 10 mg/mL** | | | | | | | | | | |
|---|---|---|---|---|---|---|---|---|---|---|
| Time Initial | 1.5 X 10⁵ | | 1.2 X 10⁵ | | 8.0 X 10⁵ | | 3.6 X 10⁵ | | 4.0 X 10⁵ | |
| 14 Days | < 10 | 5.2 | < 10 | 5.1 | < 10 | 5.8 | < 10 | 5.6 | 7.0 X 10⁴ | 0.8 |
| 28 Days | < 10 | 5.2 | < 10 | 5.1 | < 10 | 5.9 | < 10 | 5.6 | 1.6 X10³ | 2.4 |

In these batches, methylparaben and propylparaben level were zero. The results show that the test complies with the USP requirement. This shows that neramexane itself has sufficient preservative efficacy.

The same tests were run on 10 mg/mL neramexane mesylate oral solution with preservatives at different levels. The results are shown in Table 22.

**Table 22. APE Test Results**

| **Inoculum:** | **Pseudomonas aeruginosa** | | **Escherichia coli** | | **Staphylococcus aureus** | | **Candida albicans** | | **Aspergillus niger** | |
|---|---|---|---|---|---|---|---|---|---|---|
| **ATCC No.** | **ATCC 9027** | | **ATCC 8739** | | **ATCC 6538** | | **ATCC 10231** | | **ATCC 16404** | |
| | CFU /mL | Log Reduction | CFU /mL | Log Reduction | CFU /mL | Log Reduction | CFU /mL | Log Reduction | CFU /mL | Log Reduction |
| **Methylparaben : Propylparaben 0.05:0.005** | | | | | | | | | | |
| Time Initial | 1.5 X 10⁵ | | 1.2 X 10⁵ | | 8.0 X 10⁵ | | 3.6 X 10⁵ | | 4.0X10⁵ | |
| 14 Days | <10 | 5.2 | <10 | 5.1 | <10 | 5.9 | <10 | 5.6 | 2.6 X 10³ | 2.2 |
| 28 Days | < 10 | 5.2 | < 10 | 5.1 | <10 | 5.9 | < 10 | 5.6 | 4.9 X 10² | 2.9 |

| **Methylparaben : Propylparaben 0.08:0.008** | | | | | | | | | | |
|---|---|---|---|---|---|---|---|---|---|---|
| Time Initial | 1.5 X 10⁵ | | 1.2X10⁵ | | 8.0 X 10⁵ | | 3.6 X10⁵ | | 4.0 X 10⁵ | |
| 14 Days | <10 | 5.2 | <10 | 5.1 | <10 | 5.9 | <10 | 5.6 | 3.7 X 10³ | 2.0 |
| 28 Days | <10 | 5.2 | <10 | 5.1 | <10 | 5.9 | <10 | 5.6 | 1.5 X 10³ | 2.4 |

| **Methylparaben : Propylparaben 0.1:0.01** | | | | | | | | | | |
|---|---|---|---|---|---|---|---|---|---|---|
| Time Initial | 1.5 X10⁵ | | 1.2X10⁵ | | 8.0 X 10⁵ | | 3.6 X 10⁵ | | 4.0X10⁵ | |
| 14 Days | <10 | 5.2 | <10 | 5.1 | < 10 | 5.9 | <10 | 5.6 | 4.4 X 10² | 3.0 |
| 28 Days | <10 | 5.2 | < 10 | 5.1 | < 10 | 5.9 | <10 | 5.6 | 2.9 X 10² | 3.1 |

In these experiments, formulations were prepared with different levels of methylparaben: propylparaben. These were: 0.05:0.005; 0.08:0.008 and 0.1:0.01. The formulations show preservative effectiveness at all levels as they pass USP requirement.

Key embodiments of the invention include the following:

In a first embodiment, the invention provides a use as claimed in claim 1.

In a second embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof is at least about 1 mg/mL.

In a third embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof has an overall strength of about 2 mg/mL.

In a fourth embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof has an overall strength of about 4 mg/mL.

In a fifth embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof has an overall strength of about 5 mg/mL.

In a sixth embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof has an overall strength of about 10 mg/mL.

In a seventh embodiment, the invention comprises the composition for use in the first embodiment, wherein the concentration of the neramexane, its optical isomers, diastereoisomers, polymorphs, enantiomers, hydrates, pharmaceutically acceptable salts and mixtures thereof has an overall strength of about 20 mg/mL.

In an eight embodiment, the invention comprises the composition for use in the first embodiment, wherein the neramexane, its optical isomers, diastereoisomers, polymorphs, enantiomers, hydrates, pharmaceutically acceptable salts and mixtures thereof is neramexane or a pharmaceutically acceptable salt thereof.

In a ninth embodiment, the invention comprises the composition for use in the eighth embodiment, wherein the neramexane, its optical isomers, diastereoisomers, polymorphs, enantiomers, hydrates, pharmaceutically acceptable salts and mixtures thereof is neramexane mesylate.

In a tenth embodiment, the invention comprises the composition for use in the eighth embodiment, wherein the concentration of neramexane or of the pharmaceutically acceptable salt thereof is in the range from about 2 mg/mL to about 100 mg/mL.

In an eleventh embodiment, the invention comprises the composition for use in the tenth embodiment, wherein the concentration of neramexane or of the pharmaceutically acceptable salt thereof is in the range from about 5 mg/mL to about 10 mg/mL.

In a twelfth embodiment, the invention comprises the composition for use in the first embodiment, wherein the aqueous vehicle is purified water, USP.

In a thirteenth embodiment, the invention comprises the composition for use in the first embodiment, optionally comprising one or more sweeteners present in an amount ranging from about 10 mg/ml to about 500 mg/ml.

In an fourteenth embodiment, the invention comprises the composition for use in the thirteenth embodiment, wherein the sweetener is selected from the group consisting of sorbitol, sucrose, saccharin sodium, and aspartame.

In a fifteenth embodiment, the invention comprises the composition for use in the thirteenth embodiment, wherein the sweetener is sorbitol.

In a sixteenth embodiment, the invention comprises the composition for use in the first embodiment, optionally comprising a solubilizer selected from the group consisting of propylene glyco, polyethylene glycol, and glycerin present in an amount ranging from about 8% to about 12% w/v

In a seventeenth embodiment, the invention comprises the composition for use in the sixteenth embodiment, wherein the solubilizer is glycerin.

In an eighteenth embodiment, the invention comprises the composition for use in the sixteenth embodiment, wherein the glycerin is present in an amount ranging from about 8% w/v to about 12% w/v solution.

In a nineteenth embodiment, the invention comprises the composition for use in the first embodiment, further comprising one or more excipients selected from the group consisting of flavors, flavor enhancers, taste masking agents, thickeners, stabilizers, and crystallization inhibitors.

In a twentiethembodiment, the invention comprises the composition for use in the nineteenth embodiment, wherein the flavor is selected from the group consisting of natural peppermint #104, artificial cherry #10641, artificial grape #255, orange N&A 583K and artificial grape bubble gum # 998.

In a twenty-first embodiment, the invention comprises the composition for use in the nineteenth embodiment, wherein the flavor is present in a concentration ranging from about 0.05% to about 2.0%.

In a twenty-second embodiment, the invention comprises the composition for use in the first embodiment, further comprising a buffer to adjust pH of the solution.

In a twenty-third embodiment, the invention comprises the composition for use in the twenty-second embodiment, wherein the buffer is selected from the group consisting of citric acid, sodium citrate, acetic acid, sodium acetate, sodium phosphate, and combinations of two or more of the foregoing.

In a twenty-fourth embodiment, the invention comprises the composition for use in the twenty-second embodiment, wherein the buffer is a combination of citric acid and sodium citrate.

In a twenty-fifth embodiment, the invention comprises the composition for use in the twenty-second embodiment, wherein the buffer is present an amount ranging from about 1 mg/ml to about 10 mg/ml.

In a twenty-sixth embodiment, the invention comprises the composition for use in the twenty-second embodiment, wherein the pH is adjusted to between about 4.5 to about 6.5.

In a twenty-seventh embodiment, the invention comprises the composition for use in the twenty-second embodiment, wherein the pH is adjusted to about 5.5.

In a twenty-eighth embodiment, the invention comprises the composition for use in the first embodiment which is preservative free.

In a twenty-ninth embodiment, the invention comprises the composition for use in the first embodiment, further comprising a preservative, wherein the concentration of the preservative is less than the concentration required to effectively preserve the corresponding placebo composition.

In a thirtieth embodiment, the invention comprises the composition for use in the first embodiment, further comprising a preservative in an amount ranging from about 0.05% to about 2.0% w/v.

In a thirty-first embodiment, the invention comprises the composition for use in the thirtieth embodiment, further comprising a preservative in an amount ranging from about 0.1% to about 1.0% w/v.

In a thirty-second embodiment, the invention comprises the composition for use in the thirtieth embodiment, wherein the preservative is present in an amount of about 0.1 % w/v.

In a thirty-third embodiment, the invention comprises the composition for use in the twenty-ninth embodiment, wherein the preservative is selected from the group consisting of methyl paraben, propyl paraben, sodium benzoate, potassium sorbate, and combinations of two or more of the foregoing.

In a thirty-fourth embodiment, the invention comprises the composition for use in the first embodiment, comprising
a. Neramexane mesylate
b. Purified Water, USP, QS.

In a thirty-fifth embodiment, the invention comprises the composition for use in the first embodiment, comprising
a. Neramexane mesylate
b. Sorbitol solution, USP 70%
c. Citric Acid, USP
d. Sodium Citrate, USP and
e. Purified Water, USP, QS.
In a thirty-sixth embodiment, the invention comprises the composition for use in the first embodiment, comprising
a. Neramexane mesylate 0.20% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a thirty-seventh embodiment, the invention comprises the composition for use in the first embodiment, comprising
a. Neramexane HCl 0.20% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a thirty-eighth embodiment, the invention comprises the composition for use in the first embodiment, comprising
a. Neramexane mesylate 0.5% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a thirty-ninth embodiment, the invention comprises the composition for use in the first embodiment, comprising
a. Neramexane HCl 0.5% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a fortieth embodiment, the invention comprises the composition for use in the first embodiment, comprising
a. Neramexane mesylate 1.0% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a forty-first embodiment, the invention comprises the composition for use in the first embodiment, comprising
a. Neramexane HCl 1.0% w/v;
b. Sorbitol solution, USP 70%, 30.0% w/v;
c. Methylparaben, NF 0.1% w/v;
d. Propylparaben, NF 0.01 % w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS;
k. Prosweet N & A FL Enhancer, 1% w/v.
In a forty-second embodiment, the invention comprises the composition for use in the first embodiment, comprising
a. Neramexane mesylate 2.0% w/v;
b. Sorbitol solution, USP 70%, 30.0% w/v;
c. Methylparaben, NF 0.1 % w/v;
d. Propylparaben, NF 0.01% w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.
In a forty-third embodiment, the invention comprises the composition for use in the first embodiment, comprising
a. Neramexane HCl, 2.0% w/v;
b. Sorbitol solution, USP 70%, 30.0% w/v;
c. Methylparaben, NF 0.1 % w/v;
d. Propylparaben, NF 0.01 % w/v;
e. Propylene Glycol, USP, 2.50% w/v;
f. Glycerin, USP; 10.0% w/v;
g. Natural Peppermint Flavor #104 0.5% w/v;
h. Citric Acid, USP 0.19% w/v;
i. Sodium Citrate, USP 0.88% w/v;
j. Purified Water, USP, QS; and
k. Prosweet N & A FL Enhancer, 1% w/v.

## Claims

1. Use of a compound selected from neramexane, its optical isomers, diastereoisomers, polymorphs, enantiomers, hydrates, pharmaceutically acceptable salts and mixtures thereof as an antimicrobial compound in an aqueous based pharmaceutical composition comprising an aqueous vehicle, wherein the composition is preservative free or wherein the composition further comprises a preservative, wherein the concentration of the preservative is less than the concentration required to effectively preserve the corresponding placebo composition according to the USP<51 > preservative efficacy test.

2. Use as claimed in claim 1, wherein said compound is neramexane or a pharmaceutically acceptable salt thereof, preferably selected from neramexane mesylate.

3. Use as claimed in claim 1 or 2, wherein the concentration of said compound in the composition is in the range 2-100 mg/mL or in the range 5-10 mg/mL.

4. Use as claimed in any of claims 1-3, wherein said composition further comprises one or more sweeteners present in an amount in the range 10-500 mg/mL.

5. Use as claimed in claim 4, wherein said sweetener is selected from sorbitol, sucrose, saccharin sodium and aspartame.

6. Use as claimed in any of claims 1-5, wherein said composition further comprises a solubiliser selected from propylene glycol, polyethylene glycol and glycerin, said solubiliser being present in an amount in the range 8-12% w/v.

7. Use as claimed in any of claims 1-6, wherein said composition further comprises another active compound effective in the management of CNS-related conditions or disease, wherein said active compound is different to the compound as defined in claim 1.

8. Use as claimed in any of claims 1-7, wherein said composition further comprises a buffer to adjust the pH of the solution.

9. Use as claimed in claim 8, wherein the pH is adjusted to between 4.5 to 6.5 or wherein the pH is adjusted to 5.5.

10. Use as claimed in any of claims 1-9, wherein said aqueous vehicle is purified water, USP.

11. Use as claimed in any of claims 1-10, wherein water is the only liquid component of the composition.

12. Use as claimed in any of claims 1-11, wherein said composition further comprises one or more excipients selected from the group consisting of flavours, flavour enhancers, taste masking agents, thickeners, stabilisers and crystallisation inhibitors.

## Patentansprüche

1. Verwendung einer Verbindung, ausgewählt aus Neramexan, seinen optischen Isomeren, Diastereoisomeren, Polymorphen, Enantiomeren, Hydraten, pharmazeutisch annehmbaren Salzen und Gemischen davon, als antimikrobielle Verbindung in einer pharmazeutischen Zusammensetzung auf Wasserbasis, umfassend einen wässrigen Träger, wobei die Zusammensetzung frei von Konservierungsmitteln ist oder wobei die Zusammensetzung ferner ein Konservierungsmittel umfasst, wobei die Konzentration des Konservierungsmittels geringer ist als die Konzentration, die für ein wirksames Konservieren der entsprechenden Placebozusammensetzung gemäß dem USP <51> Konservierungsmittelwirksamkeitstest erforderlich ist.

2. Verwendung nach Anspruch 1, wobei die Verbindung Neramexan oder ein pharmazeutisch annehmbares Salz davon ist, vorzugsweise ausgewählt aus Neramexanmesylat.

3. Verwendung nach Anspruch 1 oder 2, wobei die Konzentration der Verbindung in der Zusammensetzung im Bereich von 2-100 mg/mL oder im Bereich von 5-10 mg/mL liegt.

4. Verwendung nach einem der Ansprüche 1 bis 3, wobei die Zusammensetzung ferner einen oder mehrere Süßstoff(e) umfasst, der bzw. die in einer Menge im Bereich von 10-500 mg/mL vorhanden ist bzw. sind.

5. Verwendung nach Anspruch 4, wobei der Süßstoff ausgewählt ist aus Sorbitol, Sucrose, Saccharinnatrium und Aspartam.

6. Verwendung nach einem der Ansprüche 1 bis 5, wobei die Zusammensetzung ferner einen Löslichmacher umfasst, ausgewählt aus Propylenglycol, Polyethylenglycol und Glycerin, wobei der Löslichmacher in einer Menge im Bereich von 8-12% w/v vorhanden ist.

7. Verwendung nach einem der Ansprüche 1 bis 6, wobei die Zusammensetzung ferner eine weitere aktive Verbindung umfasst, die in der Behandlung von Zuständen oder einer Erkrankung in Zusammenhang mit dem ZNS wirksam ist, wobei sich die aktive Verbindung von der Verbindung wie in Anspruch 1 definiert unterscheidet.

8. Verwendung nach einem der Ansprüche 1 bis 7, wobei die Zusammensetzung ferner einen Puffer zum Einstellen des pH-Wertes der Lösung umfasst.

9. Verwendung nach Anspruch 8, wobei der pH-Wert auf 4,5 bis 6,5 eingestellt ist oder wobei der pH-Wert auf 5,5 eingestellt ist.

10. Verwendung nach einem der Ansprüche 1 bis 9, wobei der wässrige Träger gereinigtes Wasser nach USP ist.

11. Verwendung nach einem der Ansprüche 1 bis 10, wobei Wasser die einzige flüssige Komponente der Zusammensetzung ist.

12. Verwendung nach einem der Ansprüche 1 bis 11, wobei die Zusammensetzung ferner einen oder mehrere Hilfsstoff(e) umfasst, ausgewählt aus der Gruppe bestehend aus Aromastoffen, Aromaverstärkern, Geschmacksmaskierungsmitteln, Verdickungsmitteln, Stabilisatoren und Kristallisationshemmern.

## Revendications

1. Utilisation d'un composé sélectionné parmi le neramexane, ses isomères optiques, diastéréoisomères, polymorphes, énantiomères, hydrates, sels pharmaceutiquement acceptables et des mélanges de ceux-ci comme composé antimicrobien dans une composition pharmaceutique à base aqueuse comprenant un véhicule aqueux, dans laquelle la composition ne contient pas d'agent conservateur ou dans laquelle la composition contient également un agent conservateur, la concentration agent conservateur étant plus basse que celle requise pour conserver efficacement la composition placebo correspondante conformément au test d'efficacité de conservation antimicrobienne USP<51>.

2. Utilisation selon la revendication 1, dans laquelle ledit composé est le neramexane ou un sel pharmaceutiquement acceptable de celui-ci qui est préférablement un mésylate de neramexane.

3. Utilisation selon la revendication 1 ou 2, dans laquelle la concentration en ledit composé dans la composition est dans la plage de 2 à 100 mg/mL ou dans la plage de 5 à 10 mg/mL.

4. Utilisation selon l'une quelconque des revendications 1 à 3, dans laquelle ladite composition comprend également un ou plusieurs édulcorants présents dans une quantité dans la plage de 10 à 500 mg/mL.

5. Utilisation selon la revendication 4, dans laquelle ledit édulcorant est sélectionné parmi le sorbitol, le saccharose, la saccharine sodique et l'aspartame.

6. Utilisation selon l'une quelconque des revendications 1 à 5, dans laquelle ladite composition comprend également un agent solubilisant sélectionné parmi le propylène glycol, le polyéthylène glycol et la glycérine, ledit agent solubilisant étant présent dans une quantité dans la plage de 8 à 12 % p/v.

7. Utilisation selon l'une quelconque des revendications 1 à 6, dans laquelle ladite composition comprend également un autre composé actif efficace dans la prise en charge de maladies ou d'affections liées au SNC, ledit composé actif étant différent du composé tel que défini dans la revendication 1.

8. Utilisation selon l'une quelconque des revendications 1 à 7, dans laquelle ladite composition comprend également un tampon pour ajuster le pH de la solution.

9. Utilisation selon la revendication 8, dans laquelle le pH est ajusté entre des valeurs allant de 4,5 à 6,5 ou dans laquelle le pH est ajusté à 5,5.

10. Utilisation selon l'une quelconque des revendications 1 à 9, dans laquelle ledit véhicule aqueux est l'eau purifiée USP.

11. Utilisation selon l'une quelconque des revendications 1 à 10, dans laquelle l'eau est le seul composant liquide de la composition.

12. Utilisation selon l'une quelconque des revendications 1 à 11, dans laquelle ladite composition comprend également un ou plusieurs excipients sélectionnés dans le groupe consistant en des arômes, des exhausteurs de goût, des agents de masquage du goût, des épaississants, des stabilisants et des inhibiteurs de la cristallisation.
